# EUROPEAN PATENT SPECIFICATION

(11) **EP 3 288 965 B1**
(45) Date of publication and mention of the grant of the patent: **26.06.2019**
(21) Application number: 16728097.3
(22) Date of filing: 26.04.2016
(51) Int. Cl.: C07K 14/715

(54) **A SOLUBLE CHIMERIC INTERLEUKIN-10 RECEPTOR AND THERAPEUTIC USE THEREOF**
UN RÉCEPTEUR CHIMERE DE L'INTERLEUKINE SOLUBLE ET SON UTILISATION THÉRAPEUTIQUE
CHIMÄRES LÖSLICHES INTERLEUKIN REZEPTOR UND THERAPEUTISCHE VERWENDUNG DAVON

(30) Priority: 29.04.2015 IT UB20150391
(43) Date of publication of application: 07.03.2018
(73) Proprietor: Mediolanum Farmaceutici S.p.A., 20143 Milano (IT)
(72) Inventor: FILACI, Gilberto, 16124 Genova GE (IT); FENOGLIO, Daniela, 16124 Genova GE (IT); FERRERA, Francesca, 16124 Genova GE (IT)
(74) Representative: Perani & Partners S.p.A.
(86) International application number: PCT/IB2016/052359
(87) International publication number: WO 2016/174575

(56) References cited:
- US-A1- 2009 111 146
- MIZUE TERAI ET AL: "Human interleukin 10 receptor 1/IgG1-Fc fusion proteins: immunoadhesins for human IL-10 with therapeutic potential", CANCER IMMUNOLOGY, IMMUNOTHERAPY, SPRINGER, BERLIN, DE, vol. 58, no. 8, 14 January 2009 (2009-01-14), pages 1307-1317, XP019706358, ISSN: 1432-0851
- KRATZ ET AL: "Albumin as a drug carrier: Design of prodrugs, drug conjugates and nanoparticles", JOURNAL OF CONTROLLED RELEASE, ELSEVIER, AMSTERDAM, NL, vol. 132, no. 3, 18 December 2008 (2008-12-18), pages 171-183, XP025714816, ISSN: 0168-3659, DOI: 10.1016/J.JCONREL.2008.05.010 [retrieved on 2008-11-29]
- MUHAMMAD SAJID HAMID AKASH ET AL: "IL-1Ra and its Delivery Strategies: Inserting the Association in Perspective", PHARMACEUTICAL RESEARCH, vol. 30, no. 11, 22 June 2013 (2013-06-22) , pages 2951-2966, XP055216825, ISSN: 0724-8741, DOI: 10.1007/s11095-013-1118-0

## Description

### FIELD OF THE INVENTION

The present invention regards a new soluble chimeric receptor for interleukin 10 (IL10) characterized by low immunogenicity capable of blocking the interaction between IL-10 and its receptor which is situated on the cells of the host and in particular useful for treating tumors as well as in treating pathologies characterized by high levels of production of interleukin 10, such as systemic lupus erythematosus (SLE).

### STATE OF THE ART

The conventional tumor therapy approaches include:
1) the extirpation of the tumor by means of surgery,
2) the destruction of the tumor by means of chemotherapy, radiotherapy and immunotherapy,

The approaches to the immunotherapy applied up to now comprise:
1) Injections of antibodies capable of mediating the lysis of the tumor cells with different mechanisms,
2) Infusions of cytokines potentially capable of activating the anti-tumor immune response,
3) Infusions of lymphocytes potentially capable of directly killing the tumor;
4) Immunization with tumor-derived cellular peptides, proteins or lysates.
5) Immunization with genes (in DNA or RNA form) which encode for the antigens associated with the tumors (TAA).
6) Inhibition of the immuno-regulatory functions through specific biological agents (anti-CTLA4, anti PD1, anti PD1-Ligand antibodies)

Nevertheless, the conventional therapies give rise to variable results, in relation to the histological type or to the tumor stage. Regarding the immunotherapy, independent of the type of therapy followed, unsatisfactory results were obtained (20-30% of the clinical responses in different clinical protocols, with the sole exception relative to the use of monoclonal antibodies in specific diseases such as B-cell lymphoma and breast tumor).

Overall, the therapeutic arsenal used up to now against tumors mainly consists of aggressive maneuvers via use of agents having the purpose of destroying cancer, which do not facilitate the normal process of immunological control which in healthy individuals prevents the appearance or the progression of tumors.

Since a complete deletion of the tumor is rarely achievable due to multiple reasons of biological nature, the surviving tumor cells remain in the body of most tumor patients, even though they are under therapy. These cells in fact maintain the capacity to evade an immune system still paralyzed both by tumor-dependent biological factors and by the aforesaid anti-tumor therapies: therefore they can induce a relapse and a progression of the tumor. Indeed, even if the tumors have aberrant genes that induce substantial modifications from a morphological, phenotypic and functional standpoint with respect to the normal autologous cells, these evade immunological surveillance. This phenomenon is even more surprising in consideration of the fact that tumors express tumor-associated antigens (TAAs) and are infiltrated by tumor-specific T-lymphocytes. Among the various known biological mechanisms that contribute to verifying the immune evasion by the tumor, the activity of the regulatory T-lymphocytes has a key role - these secrete IL10 in the tumor site. In order to neutralize the latter phenomenon, it would be necessary to provide a molecule capable of inhibiting the activity of the regulatory T-lymphocytes in the tumor site. The administration of such molecule would limit the immune evasion of the tumor and would allow the immune system of the host to recover an effective anti-tumor reactivity.

Regarding the current therapy of the systemic lupus erythematosus treatment, this consists of the administration of steroids and immunosuppressants, drugs which do not have a specificity with regard to the action mechanism and in addition they can cause serious, diverse side effects. A more recent therapy provides for the administration of Belimumab, an anti Blys human antibody, in other words a therapeutic agent capable of inhibiting the activation of the B-lymphocytes.

SLE, along with other autoimmune pathologies, is characterized by an abnormal immune response of the B-lymphocytes and by an increased secretion of IL-10 which causes a hyperproduction of autoantibodies with consequent activation of the systemic inflammatory process (Llorente et al., "In vivo production of interleukin-10 by non T-cell in rheumatoid arthritis, Sjögren' syndrome and systemic lupus erythematosus: a potential mechanism of B-lymphocyte hyperactivity and autoimmunity" Arthritis Rheum, 1994; 37: 1647-55"). It is interesting that the treatment of patients affected by SLE with an anti-IL10 monoclonal antibody had beneficial and favorable therapeutic effects. (Llorente L. et al., "Clinical and biological effects of antiinterleukin 10 monoclonal antibody administration in systemic lupus erythematosus. Arthritis Rheum.2000; 43: 1790-1800). There is therefore the need to provide a therapeutic agent capable of reducing the production of autoantibodies, for example through the neutralization of IL-10.

### BACKGROUND OF THE INVENTION

The generation of the anti-IL10 immunoadhesin, i.e. a fusion protein constituted by the portion Fc of the immunoglobulin (Ig) and by the alpha chain of the IL-10 receptor, has been previously described (Terai M. et al "Human intertleukin 10 receptor 1/ IgG1-Fc fusion proteins for human IL-10 with therapeutic potential" Cancer Immunol. Immunother. 2009 Aug.; 58(8): 1307-17 EPUB.2009 Jan 14).

This fusion protein has a series of disadvantages caused by the fact that it contains the portion Fc of the IgG1.

Indeed, the immunoglobulin can cause immune/inflammatory reactions mediated by the bond of the Fc to its receptors present on most of the cells of the immune system (antibody-dependent cellular cytotoxicity -ADCC) or to the complement (complement-dependent cytoxicity -CDC-). This can lead to collateral immune/inflammatory reactions that can have significant repercussions on the safety and tolerability of the product. (Antibody Fc: Linking Adaptive and Innate Immunity, Margaret Ackerman and Falk Nimmerjahn, Academic Press 2014; Kapur R, Einarsdottir HK, Vidarsson G: IgG-effector functions: "the good, the bad and the ugly". Immunol Lett. 2014 Aug;160(2):139-44; Karsten CM, Köhl J: The immunoglobulin, IgG Fc receptor and complement triangle in autoimmune diseases. Immunobiology. 2012 Nov; 217(11):1067-79; Dijstelbloem HM, van de Winkel JG, Kallenberg CG: Inflammation in autoimmunity: receptors for IgG revisited. Trends Immunol. 2001 Sep;22(9):510-6).

In US 20090111146, in which the inventors are some of the authors of the preceding publication, a fusion protein is described in which a constant region of the human antibody is fused with an extracellular region of the IL-10. By constant regions of the human antibody, it is intended constant regions of the IgG in particular selected from among the following classes;
a) the Fc part,
b) a region that includes CH2 and CH3,
c) a region that comprises the hinge part, CH2 and CH3,
d) a region in which CH1-CH3 are connected,
e) a region obtained by deletion, addition, substitution or insertion of 1 or several amino acids in one of the aforesaid regions a)-d) and which functions as a constant region.

Also this fusion polypeptide type can be used for correlated IL-10 diseases. In particular, the fusion proteins in which the extracellular region of the IL-10 receptor is fused with:
a1) a constant region of IgG1 in which a deletion of the hinge region has been executed, or
a2) a constant region of IgG1, having the hinge region mutated, generated by the mutation of the cysteine in the hinge region in a manner such to not form a dimer (these molecules can be employed for promoting the activation of the cytotoxic T cells).

This type of fusion proteins could be used for treating cancer, but their use is potentially subject to a high risk of induction of side effects tied to the presence of an essentially pro-inflammatory molecular portion, like the fragment Fc or the heavy changes CH1-CH3 contained in Fc (see above). The collateral and systemic immune/inflammatory reactions potentially induced by such molecules could have significant repercussions on the safety and tolerability of the product.

### OBJECTS OF THE INVENTION

Object of the present invention is therefore the generation of an inhibitor of IL-10 in particular to be used as therapeutically effective agent for all types of pathologies in which IL-10 has a pathogenic valence and which does not have the drawbacks of the above-described known inhibitors of IL-10.

Object of the present invention is also the use of such inhibitor of IL-10 in the treatment of tumors.

Further object of the present invention is the use of such inhibitor of IL-10 in the treatment of SLE.

Further object of the invention is to generate an inhibitor of IL-10 that does not have molecular portions potentially capable of inducing inflammatory/immune phenomena, such as portions of antibodies or portions of amino acid sequences not contained in human molecules (hence substantially not carrying mutations or exogenous molecules). This in order to ensure the full tolerability thereof and eliminate risks tied to the induction of side effects.

### SUMMARY OF THE INVENTION

The above-described objects of the present invention are achieved with the generation of a chimeric fusion protein albumin-IL10 receptor capable of antagonizing the bond of IL-10 to the natural receptor present on the cell surface.

The presence of albumin within the fusion polypeptide confers stability and solubility to this molecule. In addition, the molecule is little immunogenic in syngeneic individuals, so as to avoid risks of developing immune responses against the fusion protein, object of the present invention.

Further object of the present invention is the aforesaid fusion protein for use as medication, in particular for inhibiting the correlated IL-10 diseases.

Further object of the present invention is the aforesaid soluble chimeric fusion protein albumin-IL10 for use in the treatment of tumors.

Further object of the present invention is the aforesaid fusion protein for use in the treatment of SLE.

Further object of the present invention is a polynucleotide construct, preferably a gene, that encodes for this fusion protein.

Further object of the present invention is the aforesaid gene for use as medication, in particular for inhibiting the diseases mediated by IL-10.

Further object of the present invention is the aforesaid gene for use in the treatment of tumors.

Further object of the present invention is the aforesaid gene for use in the treatment of SLE.

### DESCRIPTION OF THE FIGURES

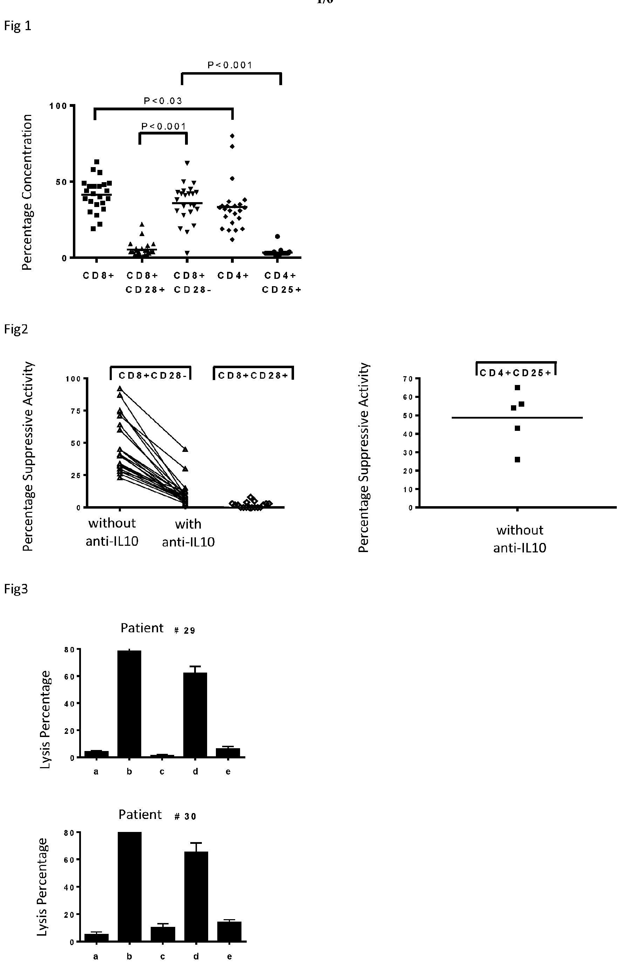

Figure 1 shows the phenotype and functional analysis of the tumor-infiltrating lymphocytes. The γ-axis shows the individual percentage concentrations of each of the subpopulations of T cells: CD8+, CD8+CD28+, CD8+CD28-, CD4+, CD4+CD25+ present in the lymphocyte population infiltrating tumors isolated from 22 patients. The figure also reports the significant differences between the average percentages.
Figure 2 reports the immunosuppression activity carried out by T cells CD8+CD28+, CD8+CD28-(left graph) infiltrating tumors isolated from 23 patients, tested in an assay of inhibition of T cell proliferation induced by an anti-CD3 monoclonal antibody (mAb). The data are expressed as percentage of inhibition of the CD3-induced proliferation activity measured through incorporation of 3H-Thymidine in the proliferating cells and reading with beta-counter and expressed as counts per minute (cpm) in the single cocultures. The graph to the right shows the immunosuppressive function of T cells CD4+CD25+ infiltrating tumors coming from 5 patients. The inhibition of the suppressive activity by a monoclonal antibody (mAb) neutralizing the biological activity of IL-10 is reported only in the case of the functional tests executed with lymphocytes TregCD8+CD28- (left graph).
Figure 3 shows the inhibition of the cytotoxic activity of a cell line CTL specific for the peptide p540 of telomerase against T2 cells pulsed with the peptide p540, in the presence or in the absence of T cells CD28+CD28-, derived from primary tumors of two patients. The cultures containing T lymphocytes CD28+CD28- were conducted in "transwell" plates in order to separate the T lymphocytes CD28+CD28- from the target cells (tumor line T2) and from the cytotoxic lymphocytes. a) CTL + non-pulsed T2 cells, b) CTL+ T2 cells pulsed with peptide p540 c) CTL+ T2 cells pulsed with peptide p540 +CD28+CD28- d) CTL+ T2 pulsed with peptide p540 +CD28+CD28- +anti-IL10mAb; e) CTL+ T2 cells pulsed with peptide p540 +CD28+CD28-+ isotype control antibody of the anti-IL10-mAb.

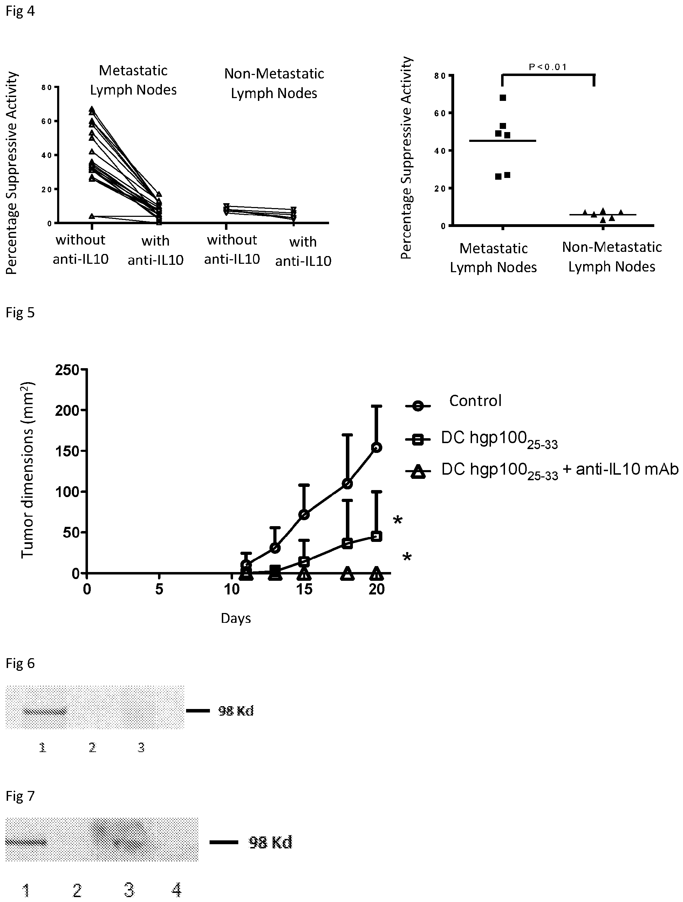

Figure 4 shows the immunosuppressive activity on the proliferation of the T cells exerted by (A) left graph: purified T cells CD28+CD28- from metastatic lymph nodes of 23 patients or from metastasis-free lymph nodes of 6 patients. (B) right graph: T cells CD24+CD25+ derived from metastatic lymph nodes of 6 patients, or from metastasis-free lymph nodes of 6 patients.
   The data are expressed as percentage of inhibition of the T cell proliferation induced by an anti-CD3 mAb, measured through incorporation of 3H-Thymidine and reading through beta-counter of the counts per minute (cpm) emitted by the cocultures.
Figure 5 reports the average area of melanoma lesions in mice respectively injected with: 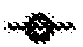 1x10⁵ B16 melanoma cells (control mice), 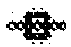 1x10⁵ B16 melanoma cells treated with dendritic cells (DC) pulsed with gp100 antigen of melanoma, inoculated through intramuscular injection (2x10⁶ DC/mouse x3 times every 7 days starting from the day of administration of the tumor). 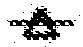 1x10⁵ B16 melanoma cells treated with DC pulsed with gp100 antigen, inoculated through intramuscular injection (2x10⁶ cells/mouse x3 times every 7 days and injected through subcutaneous injection with an anti-IL-10 mAb blocking biological activity of the cytokine (150 µg x3 times every 7 days starting from the day of administration of the tumor).
Figure 6 reports the analysis by means of Western Blot of the lysate of 293T cells transfected with: pcDNA3.1 containing the gene coding for the murine fusion protein IL10R-albumin **(lane 1),** empty pcDNA3.1 **(lane 2)** and lysate of non-transfected 293T cells **(lane 3)**
Figure 7 reports the analysis by means of Western Blot of the lysate of 293T cells transfected with pcDNA3.1 containing the gene coding for the human IL10R-albumin protein **(lane 1);** lysate of cellule 293T transfected with empty pcDNA3.1 **(lane 2);** supernatant of 293T cells transfected with pcDNA3.1 containing the gene coding for the human fusion protein IL10R-albumin **(lane 3);** supernatant of cells 293T transfected with empty pcDNA3.1 **(lane 4).**

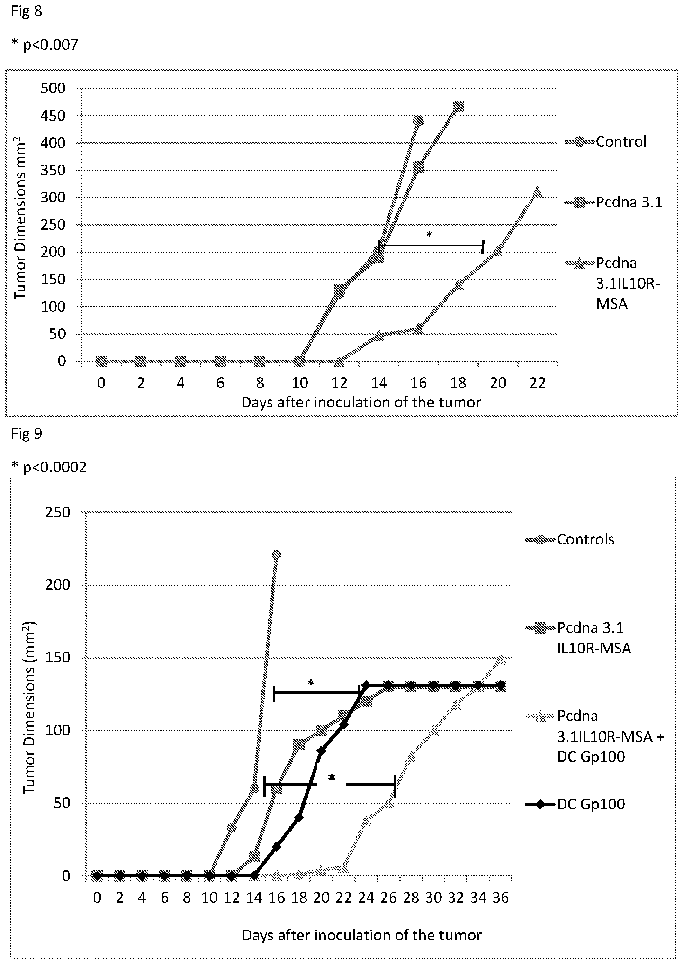

Figure 8 reports the average area of the melanoma lesions observed in mice respectively injected with:
   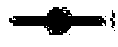 1x10⁵ B16 melanoma cells (control mice), 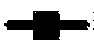 1x10⁵ B16 melanoma cells treated with a control plasmid (pcDNA 3.1) that was injected via intradermal injection (100 µg x3 times every 7 days starting from the day of administration of the tumor) .
   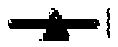 1x10⁵ B16 melanoma cells treated with a plasmid that codes for the chimeric fusion protein according to the present invention (pcDNA3.1IL10R-murin serum albumin (MSA) which has been injected intradermally (100 µg x3 times every 7 days starting from the day of administration of the tumor). The figure also reports the significant differences between the groups.
Figure 9 represents the average area of the melanoma lesions observed in mice injected with:
   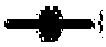 1x10⁵ B16 melanoma cells (control mice),
   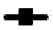 1x10⁵ B16 melanoma cells treated with a plasmid that codes for the chimeric fusion protein according to the present invention (pcDNA3.1IL10R-MSA) that was intradermally injected (100 µg x3 times every 7 days starting from the day of administration of the tumor);
   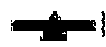 1x10⁵ B16 melanoma cells treated with a plasmid that codes for the chimeric fusion protein according to the present invention (pcDNA3.1IL10R-MSA) that was intradermally injected (100 µg x3 times every 7 days starting from the day of administration of the tumor) and intradermally injected with DC pulsed with antigen mgp100, that were injected via intramuscular injection (2x10⁶ cells/mouse x3 times 7 days, starting from the day of administration of the tumor).
   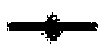 1x10⁵ B16 melanoma cells injected intradermally with DC pulsed with antigen mgp100, which were injected via intramuscular injection (2x10⁶ cells/mouse x3 times every 7 days starting from the day of administration of the tumor). The figure also reports the significant differences between the groups.

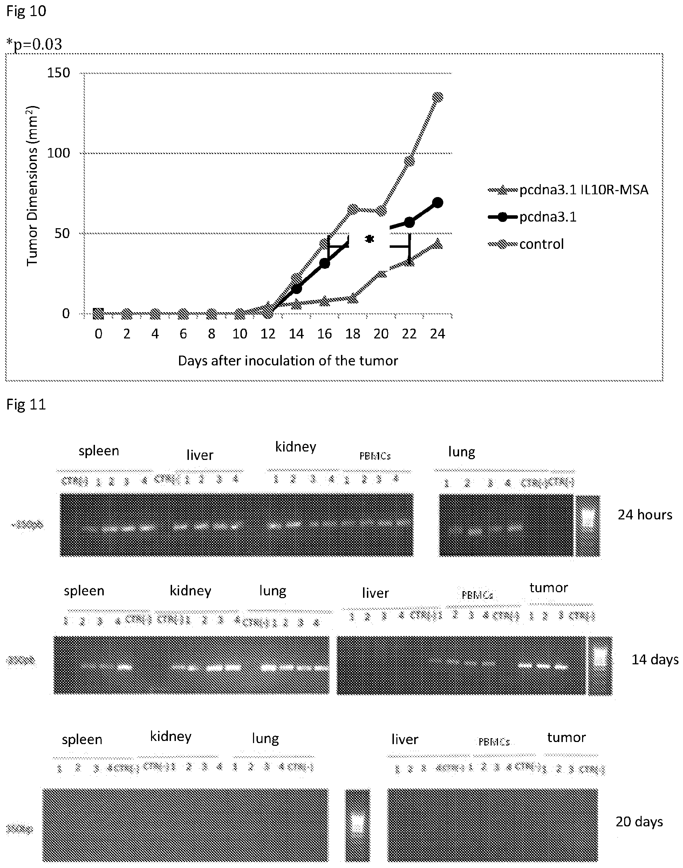

Figure 10 reports the average area of the lesions observed in mice injected with 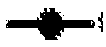 1x10⁵ cells of syngeneic bladder tumor cell lines MB49 (control), 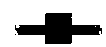 1x10⁵ of syngeneic bladder tumor MB49 treated with a control plasmid (pcDNA 3.1) that was intradermally injected (100 µg x3 times at 7 days interval between one administration and the next starting from the day of administration of the tumor). 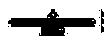 1x10⁵ syngeneic bladder tumor cell MB49 treated with a plasmid that codes for the chimeric fusion protein according to the present invention (pcDNA3.1IL10R-MSA) which was intradermally (100 µg x3 times at 7 days interval between one administration and the next starting from the day of administration of the tumor). The figure also reports the significant differences between the groups.
Figure 11 indicates the detection of the transgene- IL10R-MSA in cell DNA after having been extracted from 4x10⁶ spleen, liver, kidney and peripheral blood mononuclear cells (PBMCs) of 4 mice vaccinated with DNA (pcDNA3.1IL10R-MSA) and 1 non-vaccinated mouse (control) respectively:
   (A) 24 hours, (B) 14 days, (C) 20 days after the vaccination and after amplification with nested PCR of each of the aforesaid DNA.
Figure 12A reports, in explanatory graph form, the validation results reported in figure 12B, obtained by conducting an ELISA test on the purified human fusion protein according to the present invention; the x-axis in figure 12A reports the concentration of IL-10 and the y-axis reports the optical density.
Figure 13A reports, in explanatory form, the validation results reported in Figure 13B by means of ELISA tests conducted on the purified murine protein; the x-axis in figure 13B reports the concentration of I1L-10 and the y-axis reports the optical density.

### DETAILED DESCRIPTION OF THE INVENTION.

For the purposes of the present invention, by chimeric protein it is intended a protein deriving from the fusion of peptide sequences of multiple different proteins.

For the purposes of the present invention, by polynucleotide construct it is intended a nucleotide sequence deriving from the fusion of multiple different genes that code for multiple proteins that are different from each other.

The albumin in the chimeric fusion protein according to the present invention is preferably mammal albumin, more preferably human and murine and still more preferably human.

The fusion protein albumin - interleukin 10 receptor according to the present invention is a chimeric gene product, which is also characterized in that the albumin is bonded to the extracellular domain of the alpha chain of the IL-10 receptor.

The fact that the fusion protein, object of the invention, only contains the extracellular domain (ECD) is an advantageous aspect since this does not contain the intracytoplasmic portion of the IL-10 receptor alpha chain, i.e a portion rich in hydrophobic amino acids. Hence, the absence of the intracytoplasmic domain renders the fusion protein, object of the present invention, more stable, more soluble and obtainable with a higher yield during the productions steps (cloning) due to the limited size with respect to an analogous fusion protein containing the entire alpha chain of the IL-10 receptor. Finally, the absence of the intracytoplasmic domain renders the protein less immunogenic, thus preventing the risk that the host initiates an immune response against the fusion protein of the invention.

The receptor for interleukin 10 (IL10R) is constituted by two chains: the alpha chain that mediates the bond with the IL10 and the beta chain that transmits the signal to the cell interior. Since the object of the invention is to block the IL-10 such that it is no longer available inside the tumor microenvironment, only the alpha subunit was cloned in the chimeric construct.

The albumin is bonded to the extracellular domain of the alpha chain of the IL-10 receptor, by means of a spacer, and preferably said spacer is the hinge region of the IgG (immunogammaglobulins).

The IgG is preferably mammal IgG1, more preferably of human and murine type, still more preferably coming from lymphocytes of the peripheral blood.

The presence of the hinge region of the IgG, and preferably IgG1 confers flexibility to the portion constituted by the extracellular domain of the alpha chain of the IL 10 receptor, thus stabilizing the bond with its relative ligand (IL-10) and increasing the affinity/avidity of this interaction.

The chimeric fusion protein albumin-IL-10 receptor, object of the present invention, for use as medication, in particular for inhibiting the correlated IL-10 diseases can be parenterally administered, preferably intravenously, or even topically preferably by means of intradermal administration, subcutaneous administration etc.

Also the gene that codes for the aforesaid chimeric fusion protein albumin -interleukin 10 receptor can be employed for use as medication in particular for inhibiting the correlated IL-10 diseases and it can be parenterally administered, preferably intravenously or topically, preferably by means of intradermal administration, subcutaneous administration etc.

When the chimeric fusion protein or the relative gene in particular are employed for treating tumors, they can be parenterally administered, even topically, according to the abovementioned administration methods.

In any case, in cancer therapy, the chimeric fusion protein or the relative gene can be employed:
a) On its own, in order to increase the anti-cancer immune response. This is advisable above all in the initial stages of the disease.
b) In combination with chemotherapy and/or radiotherapy (in order to support the immune response against the tumor during a step in which the following events occurred: reduction of the tumor mass, release of a great quantity of tumor antigens by dying cells, and relapse of inflammation due to accumulation of necrotic material).
c) In combination with immunotherapeutic protocols for increasing the effectiveness of these therapeutic approaches, with the purpose of enhancing the effectiveness of endogenous immune responses through the parallel inhibition of regulatory/ suppressor cells.

In the treatment of systemic lupus erythematosus, the chimera fusion protein albumin-IL 10R according to the present invention can be administered through systemic intravenous administration on its own or in combination with the treatments of conventional type.

Reported hereinbelow are the following experimental examples, which demonstrate the key role of interleukin 10 inhibition in reducing the immunosuppressant effects of the regulatory lymphocytes which infiltrate the tumor, through the administration of the gene encoding the fusion protein according to the present invention and such protein's anti-tumor effectiveness in vivo.

### EXAMPLE 1- Demonstration of the key role of the IL-10 secreted by the tumor-infiltrating lymphocytes.

After having received informed consent from each patient, bioptic samples were obtained from a group of 42 patients affected by cancer of various origin, whose characteristics are reported in the following table 1, with whom a series of experiments was conducted adapted to demonstrate the key role of the tumor-infiltrating regulatory T-lymphocytes and of the IL-10 cytokine released thereby in the tumor microenvironment.

**TABLE 1 -SUMMARY OF THE CLINICAL CHARACTERISTICS AND DEFINITION OF THE BIOLOGICAL SAMPLES OBTAINED FROM EACH PATIENT**

| Patient N° | Cancer | Clinical state | Survival (months) | Primary tumor lesions | Metastatic lymph node* | Metastasis-free lymph node* | PBMC* |
|---|---|---|---|---|---|---|---|
| 1 | Stomach | T4 | >12 | - | + | - | + |
| 2 | Pancreas | T4 | >12 | - | + | - | + |
| 3 | Colon-Rectum | T4 | <12 | + | + | - | + |
| 4 | Pancreas | T4N2 | <12 | - | + | - | + |
| 5 | Colon-Rectum | T4 | >12 | - | + | - | + |
| 6 | Colon-Rectum | T4N1 | >12 | + | + | - | - |
| 7 | Stomach | T3 | >12 | + | + | - | + |
| 8 | Sarcoma | T4 | <12 | - | + | - | + |
| 9 | Kidney | T4N0 | >12 | - | + | + | + |
| 10 | Kidney | T1a | >12 | + | + | - | + |
| 11 | Colon-Rectum | T4 | <12 | + | + | + | + |
| 12 | Colon-Rectum | T4 | <12 | + | + | - | + |
| 13 | Colon-Rectum | T4 | >12 | + | - | + | + |
| 14 | Colon-Rectum | T4 | >12 | + | - | - | + |
| 15 | Head-Neck | T4 | <12 | - | + | - | + |
| 16 | Thyroid | T2N2 | >12 | + | + | - | + |
| 17 | Colon-Rectum | T4N0 | - | - | + | - | + |
| 18 | Colon-Rectum | T4N2 | <12 | + | + | - | + |
| 19 | Stomach | T2bN3 | >12 | + | + | - | + |
| 20 | Colon-Rectum | T3N0 | <12 | + | - | - | + |
| 21 | Stomach | T4N3 | <12 | + | + | + | + |
| 22 | Colon-Rectum | T4 | >12 | + | + | - | + |
| 23 | Hodgkin | Stage 2 | >12 | - | - | + | + |
| 24 | Melanoma | T4 | <12 | - | + | - | + |
| 25 | Colon-Rectum | T4N1 | <12 | + | + | - | + |
| 26 | Ovary | T1N0 | >12 | + | + | + | + |
| 27 | Esophagus | T3N1M1 | >12 | + | - | - | + |
| 28 | Ovary | T3M1 | <12 | + | - | - | + |
| 29 | Colon-Rectum | T4 | <12 | + | - | - | + |
| 30 | Colon-Rectum | T4 | <12 | + | - | - | + |
| 31 | Breast | T4 | - | + | - | - | + |
| 32 | Colon-Rectum | T4 | - | + | + | - | + |
| 33 | Lung | T3 | - | - | + | - | - |
| 34 | Hodgkin | Stage 2 | - | - | + | - | - |
| 35 | Prostate | T3 | - | - | + | + | - |
| 36 | Hodgkin | Stage 3 | - | - | + | - | - |
| 37 | Lung | T3 | - | - | + | - | - |
| 38 | Head-Neck | T4 | - | - | + | + | - |
| 39 | Seminoma | T1 | - | - | + | - | - |
| 40 | Lung | T2 | - | - | + | - | - |
| 41 | Neuro-Endocrine Carcinoma | T3 | - | - | + | - | - |
| 42 | Non-Hodgkin | Stage 4 | - | + | - | - | + |

| | | | | | | | |
|---|---|---|---|---|---|---|---|
| *: biological material +: available -: not available | | | | | | | |

### Example 1A: Characterization of the populations of tumor-infiltrating regulatory T-lymphocytes derived from oncological patients.

A phenotype and functional analysis was conducted of the tumor-infiltrating cells derived from bioptic samples drawn from 22 patients whose characteristics are reported in Table 1. For such purpose, the tumor samples were finely fragmented by using suitable sterile filters. Subsequently, the obtained cell suspensions were stratified and centrifuged on Ficoll gradient. Finally, the different lymphocyte subpopulations were purified through immunomagnetic "sorting" procedures by using suitable magnetic balls conjugated with specific antibodies (Miltenyi Biotech). The immunophenotype of the infiltrating lymphocytes executed with anti CD4, anti CD8, anti CD25, anti CD28 monoclonal antibodies conjugated with fluorochromes. (BD Biosciences) made possible the characterization of two populations of regulatory T lymphocytes (Treg) i.e. Treg lymphocytes CD4+CD25+ and CD8+CD28- from among the tumor-infiltrating lymphocytes, as reported in Figure 1.

### Example IB Verification of the immunosuppressive activity of the tumor-infiltrating cells on T lymphocytes.

The immunosuppressive activity of the tumor-infiltrating T cells was measured in an assay of inhibition of the proliferation of peripheral blood T lymphocytes activated with anti-CD3 mAb antibody and evaluated through incorporation of the 3H-Thymidine proliferating cells and measured in beta-counter reading as counts per minute (cpm). The data is expressed in Figure 2 as percentage of inhibition of the proliferation of T cells in the presence of anti-CD3 mAb. In particular, the test showed suppressive activity of the proliferation by T cells CD8+CD28-, but not by tumor-infiltrating T cells CD8+CD28+, drawn from 23 patients (Figure 2, left graph) and by tumor-infiltrating T cells CD4+CD25+ coming from 5 patients (Figure 2, right graph). Such suppressive activity of the tumor-infiltrating Treg lymphocytes CD8+CD28- is blocked in the presence of the anti-IL10 mAb monoclonal antibody (Figure 2, left graph).

### EXAMPLE 1-C Inhibitory activity of the T cells CD8+CD28- on the cytotoxic activity of the tumor-specific lymphocytes T.

The regulatory function of the cell population T CD8+CD28- derived from oncological samples was also evaluated regarding the cytotoxic capacity of a human cell line CTL specific for the peptide p540 of telomerase. For such purpose, the cytotoxic activity of this line CTL was tested against cells belonging to the T2 lymphoblast tumor line pulsed with the peptide p540 in the presence or in the absence of T reg cells CD8+CD28- isolated from primary tumor masses of two patients affected by HLA-A2-positive prostate tumor. The co-cultures in the presence of the intratumoral Treg lymphocytes CD28+CD28- were conducted in "transwell" plates suitable for physically separating the Treg lymphocytes from the target cells of the T2 tumor lines and from the CTL p540-specific lymphocytes. The following co-cultures were then carried out:
a) CTL + non-pulsed T2 target cells, b) CTL+ T2 target cells pulsed with peptide p540 c) CTL+ T2 target cells pulsed with peptide p540 + intratumoral Treg CD28+CD28-**d**) CTL+ T2 target cells pulsed with peptide p540 + intratumoral Treg CD28+CD28- +anti-IL10mAb; e) CTL+ T2 target cells pulsed with peptide p540 + intratumoral Treg CD28+CD28 + -mAb of isotype control with insignificant specificity.

The results of this experiment reported in Figure 3 are expressed as percentage of inhibition of the cytotoxic activity and indicate that the population Treg CD8+CD28- exerts an inhibitory activity also against the cytotoxic function of tumor-specific T cells.

### EXAMPLE ID reduction of the immunosuppressive activity by anti IL-10 mAb monoclonal antibody

The immunosuppressive activities of the population of tumor-infiltrating Treg cells are opposed by anti-IL10 monoclonal antibodies, hence demonstrating to be strictly dependent on the secretion of this cytokine. The accumulation of Treg CD8+CD28- and Treg CD4+CD25+ seems to be strictly tumor-dependent since it is only verified where the infiltration of the tumor is present both in the site of the primary tumor and in the sites of the metastasis. Indeed, only the metastatic lymph nodes, and not those free of metastasis, were found to be infiltrated by the aforesaid populations of regulatory T cells. (see figure 4)

### EXAMPLE 1-E In vivo verification of the immunosuppressive activity of the IL-10.

C57 black mice subcutaneously injected with 1 x 10⁵ cells of B16 syngeneic melanoma develop a very aggressive melanoma characterized by devastating local invasion and metastatic spreading via contiguity with the abdominal visceral organs, if the injection occurs in the abdominal area. For the purpose of identifying an effective immunotherapy, different strategies were conducted comprising:
a) 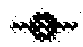 gene vaccination with plasmids that code for the melanoma-associated human or murine antigen gp100;
b) 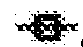 subcutaneous vaccination with immunogenic peptides derived from human gp100 (hgp100₂₅₋₃₃) or murine (mgp100₂₅₋₃₃) in the presence of adjuvant CpG (cytosine-phosphate-guanine),
c) 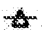 vaccination by dendritic cells (DC) preloaded with the same immunogenic peptides (gp100).

Both in a syngeneic and xenogeneic context, the vaccination with dendritic cells pulsed with the peptide gp100 resulted the most protective treatment that induced >50% reduction of the tumor mass. In a subsequent experiment, the mice subjected to the "challenge" with B16 melanoma cells were immunized according to the protocol (c) in association with the administration of an anti-IL10 mAb: such strategy was effective in inhibiting the entire tumor growth in 100% of the treated mice, as reported in Figure 5.

Since the preceding studies had already demonstrated that IL-10 causes the intratumoral differentiation of the tolerogenic dendritic cells, capable of inducing further regulatory T cells (Guiducci et al., Cancer Res. 2005; 65;3437-3446), in its entirety this data supports the innovative idea that the IL10 has an important role in determining the evasion of the tumor from immune surveillance and that the strategies aimed to block the effects of the intratumoral IL-10 at the functional/molecular level can be effective approaches for the treatment of tumors.

### EXAMPLE 2 PRODUCTION OF THE CONSTRUCTS OF THE FUSION PROTEINS:

- **HUMAN ALBUMIN** - **HINGE REGION OF HUMAN IgG1 -EXTRACELLULAR DOMAIN OF THE ALPHA CHAIN OF THE HUMAN IL 10 RECEPTOR.**
- **MURINE ALBUMIN- HINGE REGION OF MURINE IgG1 -EXTRACELLULAR DOMAIN OF THE ALPHA CHAIN OF THE MURINE IL 10 RECEPTOR**

### EXAMPLE 2.1- Preparation of the plasmid construct pcDNA 3.1 human/murine albumin - hinge region of human/murine IgG1 -extracellular domain of the alpha chain of the human/murine IL 10 receptor.

The expression plasmid pcDNA -V5-His (Life Technologies), which was employed for the study, contains the promoter of the genes of the cytomegalovirus (CMV) and the fragment of polyadenylation SV40 required for terminating the transcription and translation; in addition, it also contains the epitope V5 and a His tag useful for the evaluation and purification of the expression of the gene product. The stable selection of clones in eukaryotic cells is possible due to the presence of the gene of the resistance to G418. Two fusion proteins, respectively one human and one murine, have been engineered by bonding the cDNA of the extracellular domain (ECD) of the alpha chain of the interleukin 10 receptor derived from PBMC, respectively human and murine, to clones of respectively human and murine serum albumin cDNA acquired from ATCC. The two cDNA were bonded by means of only the hinge region of the respectively human and murine IgG1 derived from PBMCs. The cloning was carried out by using the following strategy: the cDNA of the extracellular domain (ECD) of the alpha chain of the murine interleukin 10 receptor was cloned by PCR by using the following pair of primers with the restriction sites inserted: mIL10R-KpnI for 5' - TTAGGTACCATGTTGTCGCGTTTGCTCC- 3' and mIL10R NotI rev 5'-GCGGCCGCCTGTACATATGCAAGGCTTACAACC- 3', the cDNA of the murine serum albumin was cloned by PCR by using the following pair of primers with the restriction sites inserted: MSA-NotI for 5'AAGGAAAAAAGCGGCCGCGAAGCACACAAGAG 3' and MSA-XbaI rev 5' GCTCTAGAGGCTAAGGCGTCTTTG-3'. The cDNA of the ECD of the alpha chain of the human interleukin 10 receptor was cloned by PCR by using the following pair of primers with the restriction sites inserted: hIL10R-KpnI for 5' -GGTACCATGCTGCCGTGCCTCGTAG 3' and hIL10R NotI rev 5'- GCGGCCGC TGGGCATGTGTGAGTTTTGTCACAA and the cDNA of the human serum albumin was cloned by PCR by using the following pair of primers with the restriction sites inserted: HSA-NotI for 5'-GCGGCCGCGGATGCACACAAGAGTG-3' and HSA-ApaI rev 5' GGGCCCTTATAAGCCTAAGGCA-3' . The PCR was executed on the Biorad T100 instrument.

In order to confirm the exact alignment of the two genes, the chimeric construct was sequenced with automatic sequencer (ABI 3100, Applied Biosystem)

### EXAMPLE 2.2 analysis and characterization of the murine and human chimeric fusion proteins of the invention

Subsequently, the gene products were analyzed and evaluated by means of Western Blot analysis according to the following operating modes: 293T or HEK293 cells were transfected with the two plasmids respectively murine pcDNA3.1 IL10R-albumin and human pcDNA3.1 IL10R-albumin. The relative lysates and supernatants from the aforesaid cell cultures were analyzed by means of 12.5% SDS-PAGE gel in reducing conditions and analyzed via Western blot, by employing antibodies specific for the gene products (e.g. murine anti-CD210 monoclonal antibody and human anti-IL10R alpha monoclonal antibody, murine / human anti albumin monoclonal antibody).

Cell lysates and supernatants of non-transfected cells and/or transfected with "empty" plasmid (i.e. not containing the chimeric gene) were analyzed in parallel as negative controls: as expected, no presence of the chimeric product was encountered herein. The results of this analysis are respectively reported in figures 6 and 7. Figure 6 shows the presence of the band of the expected molecular weight equal to 98 Kd in the lane containing the cell lysate of 293T cells transfected with the plasmid pcDNA3.1 containing the murine gene IL10R-albumin, but not in the lanes where the control lysates were made to run. Figure 7 shows the presence of the band of the expected molecular weight equal to 98 Kd in the lanes containing the cell lysate or the supernatant of 293T cells transfected with the plasmid pcDNA3.1 containing the human gene IL10R-albumin, but not in the lanes where the control lysate or the control supernatant were made to run. Overall, such data confirms the presence of the chimeric protein, respectively murine and human, object of the invention, in the lysate and in the supernatant of the cells transfected with the plasmids containing the chimeric genes (respectively human and murine).

### EXAMPLE 3 PRELIMINARY IN VIVO STUDIES ON THE ANTI-TUMOR ACTIVITY OF THE CHIMERIC FUSION PROTEIN ALBUMIN-EXTRACELLULAR DOMAIN OF THE ALPHA CHAIN OF THE IL 10 RECEPTOR.

### Example 3.1 - In vivo anti-tumor activity against melanoma induced by B16 melanoma tumor cells by the construct pcDNA 3.1- murine albumin- hinge region of murine IgG1 - extracellular domain of the alpha chain of the murine IL 10 receptor

3 groups of C57 black mice were respectively injected with:
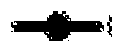 1x10⁵ B16 melanoma cells (control mice); 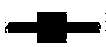 1x10⁵ B16 melanoma cells treated with a control plasmid (pcDNA 3.1) that was intradermally injected (100 µg x3 times at 7 days interval between one administration and the next, starting from the day of administration of the tumor);
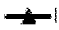 1x10⁵ B16 melanoma cells treated with a plasmid that codes for the chimeric fusion protein according to the present invention (pcDNA3.1IL10R-MSA) which was intradermally injected (100 µg x3 times at 7 days interval between one administration and the next, starting from the day of administration of the tumor);

In the course of the experiment, the areas of the tumor lesions were monitored and the animals were sacrificed, in respect of ethical norms, when the greater diameter of the neoplastic mass reached 2 cm dimensions. The results are reported in Figure 8. As inferred from this figure, the administration of the empty plasmid did not induce any significant difference with respect to the untreated control group (survival times <2 weeks). On the contrary, the mice immunized with the plasmid construct that codes for the chimeric fusion protein according to the present invention showed a drastic and significant change of the melanoma growth curve, so as to have survival times 20 days greater than those of the control mice.

### Example 3.2 In vivo anti-tumor activity, following administration of B16 melanoma tumor cells, of the plasmid construct pcDNA 3.1- murine albumin- hinge region of murine igG1 - extracellular domain of the alpha chain of the murine IL 10 receptor associated with immunization with dendritic cells pulsed with peptide mgp100.

4 groups of C57 black mice were respectively treated with:
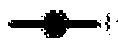 1x10⁵ of B16 melanoma cells (control mice);
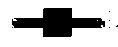 1x10⁵ of B16 melanoma cells and administration of the plasmid which codes for the chimeric fusion protein according to the present invention (pcDNA3.1IL10R-MSA) which was intradermally injected (100 µg x3 times at 7 days interval between one administration and the next, starting from the day of administration of the tumor);
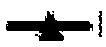 1x10⁵ of B16 melanoma cells and administration of the plasmid which codes for the chimeric fusion protein according to the present invention (pcDNA3.1IL10R-MSA) which was intradermally injected (100 µg x3 times at 7 days interval between one administration and the next, starting from the day of administration of the tumor) associated with the intradermal inoculation of DC (dendritic cells) pulsed with peptide mgp100₂₅₋₃₃, which were injected intramuscularly (2x10⁶cells/ mouse x3 times at 7 days interval between one administration and the next, starting from the day of administration of the tumor);
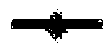 1x10⁵ of B16 melanoma cells and intradermal administration of DC (dendritic cells) pulsed with peptide mgp100₂₅₋₃₃, which were intramuscularly injected (2x10⁶cells/ mouse x3 times at 7 days interval between one administration and the next, starting from the day of administration of the tumor);
   In the course of the experiment, the areas of the tumor lesions were monitored and the animals were sacrificed, in respect of ethical norms, when the greater diameter of the neoplastic mass reached 2 cm dimensions. The results are reported in figure 9. From this data, it results that the mice treated with the plasmid construct which codes for the fusion protein, object of the invention, have a tumor growth curve that is practicable superimposable on that obtained following treatment with dendritic cells pulsed with peptide mgp100₂₅₋₃₃. This is of great importance, since the vaccination protocol with dendritic cells has proven the most effective from among the immunotherapeutic treatments against B16 melanoma (see above, Example 1-E on page 15): therefore, the treatment with the plasmid construct that codes for the fusion protein, object of the invention, has shown effectiveness equal to the best of the immunotherapeutic treatments. In addition, the mice treated with the plasmid construct that codes for the fusion protein, object of the present invention, in association with the administration of dendritic cells pulsed with peptide mgp100₂₅₋₃₃ showed a further significant slowing of the neoplastic growth curve since the appearance of appreciable lesions was delayed by a further week with respect to the animals treated with only the administration of dendritic cells pulsed peptide mgp100₂₅₋₃₃ or only with the plasmid coding for the fusion protein, object of the invention.

### Example 3.3. In vivo anti-tumor activity against bladder tumor cell line M49 of the plasmid construct pcDNA 3.1- murine albumin - hinge region of murine IgG1 - extracellular domain of the alpha chain of the murine IL 10 receptor.

3 groups of C57 black mice were respectively treated with:
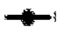 1x10⁵ cells of syngeneic bladder tumor cell lines MB49 (control),
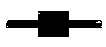 1x10⁵ syngeneic bladder tumor cell lines MB49 and administration of the "empty" control plasmid (pcDNA 3.1) that was intradermally injected (100 µg x3 times at 7 days interval between one administration and the next, starting from the day of administration of the tumor)
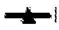 1x10⁵ syngeneic bladder tumor cell lines MB49 and administration of the plasmid which codes for the chimeric fusion protein according to the present invention (pcDNA3.1IL10R-MSA) which was intradermally injected (100 µg x3 times at 7 days interval between one administration and the next, starting from the day of administration of the tumor)
   In the course of the experiment, the areas of the tumor lesions were monitored and the animals were sacrificed, in respect of ethical norms, when the greater diameter of the neoplastic mass reached 2 cm dimensions. The results are reported in figure 10.

As inferred from this figure, the data obtained with the tumor cell lines M49 reproduce the data obtained by using the melanoma cells. Indeed, the administration of the plasmid empty did not induce any significant different with respect to the untreated control group (survival times <2 weeks). On the contrary, the mice immunized with the plasmid construct which codes for the chimeric fusion protein according to the present invention showed a drastic and significant change of the melanoma growth curve so as to have survival times 20 days greater than those of the control mice.

### EXAMPLE 4-Detection of the transgenic B lymphocytes

For the purpose of evaluating the persistence and tissue distribution of the transgene, the cell DNA was extracted from the PBMCs and from the organs (spleen, kidney, liver, lung) of 4 vaccinated mice with high reperfusion speed, intravenously with polynucleotide construct that encodes murine albumin- murine hinge region of murine IgG1 -extracellular domain of the alpha chain of the murine IL 10 receptor and of 1 non-vaccinated mouse (control) 24 hours, 14 days and 20 days after vaccination. The results are reported in Figure 11. As inferred from such figure, at day 20 after its inoculation the transgene was no longer detectable, demonstrating that its half-life inside the organism is about 2 weeks.

### EXAMPLE 5-Purification of the human chimeric protein according to the present invention.

The two human and murine chimeric proteins, object of the present invention, were purified from the supernatant of HEK293 and EXPI -293 cells, transfected with the plasmid that encodes the human and murine fusion protein.

The two human and murine proteins were validated by conducting the ELISA test respectively on two separate batches, for human proteins, and on three separate batches for the murine proteins. The ELISA test conducted on the human chimeric protein demonstrated that the human chimeric protein specifically recognizes the human IL-10 and it is in turn recognized by an anti albumin human antibody marked with HRP (radish peroxidase).

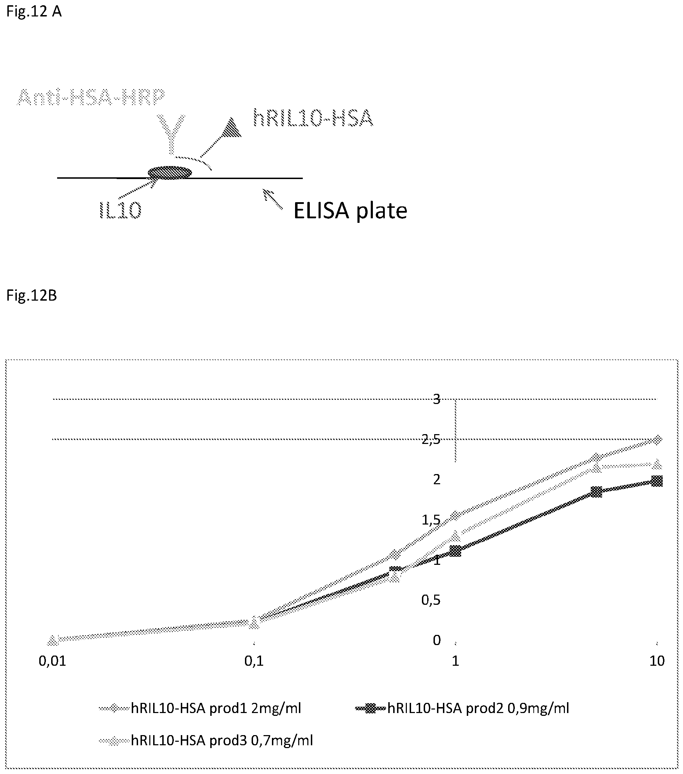

The results of such test are respectively reported in figures 12A and 12B.

This experiment demonstrates that the protein produced by the transfected cells is a chimeric protein having receptor capacity specific for IL-10 associated with an albumin structure.

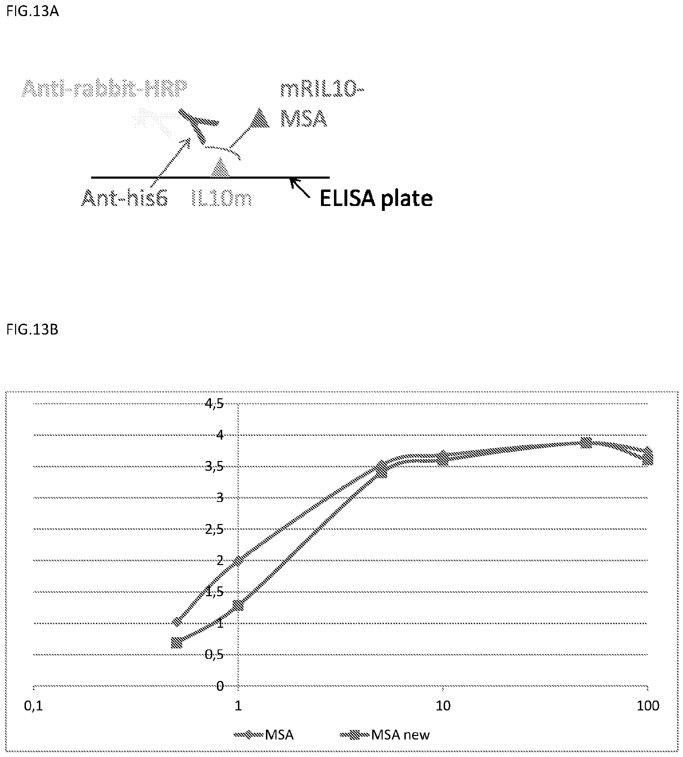

Analogously, the results obtained with ELISA test on the murine fusion protein reported in Figures 13A and 13 B demonstrated that the murine protein specifically bonds the murine interleukin, confirming the receptor effectiveness. In this case, the detection was conducted through an anti-histidine antibody marked with HRP, since the murine protein has a histidine code.

Reported hereinbelow are the sequences of the human and murine fusion protein according to the present invention relative to the fragments of the ECD domain of the alpha unit of the IL10 interceptor, of the hinge region of the immunogammaglobulin, of the albumin and of the fragments of the corresponding polynucleotide constructs.

### Sequence

<213> OrganismName : human
<400> PreSequenceString : 
MLPCLVVLLA ALLSLRLGSD AHGTELPSPP SVWFEAEFFH HILHWTPIPN QSESTCYEVA 60

LLRYGIESWN SISNCSQTLS YDLTAVTLDL YHSNGYRARV RAVDGSRHSN WTVTNTRFSV 120

DEVTLTVGSV NLEIHNGFIL GKIQLPRPKM APANDTYESI FSHFREYEIA IRKVPGNFTF 180

THKKVKHENF SLLTSGEVGE FCVQVKPSVA SRSNKGMWSK EECISLTRQY FTVTNSRV 238
<212> Type : PRT
<211> Length : 238
   Sequence Name : human IL10 R alpha extracellular domain

### Sequence

<213> Organism Name : human
<400> Pre Sequence String :
   EPKSCDKTHT CPAAA 15
<212> Type : PRT
<211> Length : 12
   SequenceName : Human HINGE

### Sequence

<213> Organism Name : human
<400> Pre Sequence String : 
 60

 120

 180

 240

 300

 360

 420

 480

 540

 585
<212> Type : PRT
<211> Length : 585
   Sequence Name : Human albumin domain

### Sequence

<213> Organism Name : human
<400> Pre Sequence String : 
atgctgccgt gcctcgtagt gctgctggcg gcgctcctca gcctccgtct tggctcagac 60

gctcatggga cagagctgcc cagccctccg tctgtgtggt ttgaagcaga atttttccac 120

cacatcctcc actggacacc catcccaaat cagtctgaaa gtacctgcta tgaagtggcg 180
 
ctcctgaggt atggaataga gtcctggaac tccatctcca actgtagcca gaccctgtcc 240

tatgacctta ccgcagtgac cttggacctg taccacagca atggctaccg ggccagagtg 300

cgggctgtgg acggcagccg gcactccaac tggaccgtca ccaacacccg cttctctgtg 360

gatgaagtga ctctgacagt tggcagtgtg aacctagaga tccacaatgg cttcatcctc 420

gggaagattc agctacccag gcccaagatg gcccccgcga atgacacata tgaaagcatc 480

ttcagtcact tccgagagta tgagattgcc attcgcaagg tgccgggaaa cttcacgttc 540

acacacaaga aagtaaaaca tgaaaacttc agcctcctaa cctctggaga agtgggagag 600

ttctgtgtcc aggtgaaacc atctgtcgct tcccgaagta acaaggggat gtggtctaaa 660

gaggagtgca tctccctcac caggcagtat ttcaccgtga ccaactctag agtt 714
<212> Type : DNA
<211> Length : 714
   Sequence Name : Nucleotide of human IL10 R alpha extracellular domain

### Sequence

<213> Organism Name : human
<400> Pre SequenceString :
   gagcccaaat cttgtgacaa aactcacaca tgcccagcgg ccgcg 45
<212> Type : DNA
<211> Length : 45
   Sequence Name : Nucleotide of human HINGE

### Sequence

<213> Organism Name : mouse
<400> Pre Sequence String : 
MLSRLLPFLV TISSLSLEFI AYGTELPSPS YVWFEARFFQ HILHWKPIPN QSESTYYEVA 60

LKQYGNSTWN DIHICRKAQA LSCDLTTFTL DLYHRSYGYR ARVRAVDNSQ YSNWTTTETR 120

FTVDEVILTV DSVTLKAMDG IIYGTIHPPR PTITPAGDEY EQVFKDLRVY KISIRKFSEL 180
 
KNATKRVKQE TFTLTVPIGV RKFCVKVLPR LESRINKAEW SEEQCLLITT EQYFTVTNLS 240

IKLI 244
<212> Type : PRT
<211> Length : 244
   Sequence Name : Mouse IL10 R alpha extracellular domain

### Sequence

<213> Organism Name : mouse
<400> Pre Sequence String :
   VPRDCGCKPC ICTGGR 16
<212> Type : PRT
<211> Length : 16
   Sequence Name : mouse hinge

### Sequence

<213> Organism Name : mouse
<400> Pre Sequence String : 
EAHKSEIAHR YNDLGEQHFK GLVLIAFSQY LQKCSYDEHA KLVQEVTDFA KTCVADESAA 60

NCDKSLHTLF GDKLCAIPNL RENYGELADC CTKQEPERNE CFLQHKDDNP SLPPFERPEA 120

EAMCTSFKEN PTTFMGHYLH EVARRHPYFY APELLYYAEQ YNEILTQCCA EADKESCLTP 180

KLDGVKEKAL VSSVRQRMKC SSMQKFGERA FKAWAVARLS QTFPNADFAE ITKLATDLTK 240

VNKECCHGDL LECADDRAEL AKYMCENQAT ISSKLQTCCD KPLLKKAHCL SEVEHDTMPA 300

DLPAIAADFV EDQEVCKNYA EAKDVFLGTF LYEYSRRHPD YSVSLLLRLA KKYEATLEKC 360

CAEANPPACY GTVLAEFQPL VEEPKNLVKT NCDLYEKLGE YGFQNAILVR YTQKAPQVST 420

PTLVEAARNL GRVGTKCCTL PEDQRLPCVE DYLSAILNRV CLLHEKTPVS EHVTKCCSGS 480

LVERRPCFSA LTVDETYVPK EFKAETFTFH SDICTLPEKE KQIKKQTALA ELVKHKPKAT 540

AEQLKTVMDD FAQFLDTCCK AADKDTCFST EGPNLVTRCK DALA 584
<212> Type : PRT
<211> Length : 584
   Sequence Name : Murine Albumin domain
   Sequence Description :

### Sequence

<213> Organism Name : mouse
<400> PreSequence String : 
atgttgtcgc gtttgctccc attcctcgtc acgatctcca gcctgagcct agaattcatt 60

gcatacggga cagaactgcc aagcccttcc tatgtgtggt ttgaagccag atttttccag 120

cacatcctcc actggaaacc tatcccaaac cagtctgaga gcacctacta tgaagtggcc 180

ctcaaacagt acggaaactc aacctggaat gacatccata tctgtagaaa ggctcaggca 240

ttgtcctgtg atctcacaac gttcaccctg gatctgtatc accgaagcta tggctaccgg 300

gccagagtcc gggcagtgga caacagtcag tactccaact ggaccaccac tgagactcgc 360

ttcacagtgg atgaagtgat tctgacagtg gatagcgtga ctctgaaagc aatggacggc 420

atcatctatg ggacaatcca tcccccaggg cccacgataa cccctgcagg ggatgagtac 480

gaacaagtct tcaaggatct ccgagtttac aagatttcca tccggaagtt ctcagaacta 540

aagaatgcaa ccaagagagt gaaacaggaa accttcaccc tcacggtccc cataggggtg 600

agaaagtttt gtgtcaaggt gctgccccgc ttggaatccc gaattaacaa ggcagagtgg 660

tcggaggagc agtgtttact tatcacgacg gagcagtatt tcactgtgac caacctgagc 720

atcaagctta tt 732
<212> Type : DNA
<211> Length : 732
   Sequence Name : Nucleotide of mouse IL10 R alpha extracellular domain

### Sequence

<213> OrganismName : mouse
<400> PreSequenceString :
   gtgcccaggg attgtggttg taagccttgc atatgtacag gcggccgc 48
<212> Type : DNA
<211> Length : 48
   Sequence Name : Nucleotide of mouse hinge

### Sequence

<213> OrganismName : mouse
<400> PreSequenceString : 
gaagcacaca agagtgagat cgcccatcgg tataatgatt tgggagaaca acatttcaaa 60

ggcctagtcc tgattgcctt ttcccagtat ctccagaaat gctcatacga tgagcatgcc 120

aaattagtgc aggaagtaac agactttgca aagacgtgtg ttgccgatga gtctgccgcc 180

aactgtgaca aatcccttca cactcttttt ggagataagt tgtgtgccat tccaaacctc 240

cgtgaaaact atggtgaact ggctgactgc tgtacaaaac aagagcccga aagaaacgaa 300

tgtttcctgc aacacaaaga tgacaacccc agcctgccac catttgaaag gccagaggct 360

gaggccatgt gcacctcctt taaggaaaac ccaaccacct ttatgggaca ctatttgcat 420

gaagttgcca gaagacatcc ttatttctat gccccagaac ttctttacta tgctgagcag 480

tacaatgaga ttctgaccca gtgttgtgca gaggctgaca aggaaagctg cctgaccccg 540

aagcttgatg gtgtgaagga gaaagcattg gtctcatctg tccgtcagag aatgaagtgc 600

tccagtatgc agaagtttgg agagagagct tttaaagcat gggcagtagc tcgtctgagc 660

cagacattcc ccaatgctga ctttgcagaa atcaccaaat tggcaacaga cctgaccaaa 720

gtcaacaagg agtgctgcca tggtgacctg ctggaatgcg cagatgacag ggcggaactt 780

gccaagtaca tgtgtgaaaa ccaggcgact atctccagca aactgcagac ttgctgcgat 840

aaaccactgt tgaagaaagc ccactgtctt agtgaggtgg agcatgacac catgcctgct 900

gatctgcctg ccattgctgc tgattttgtt gaggaccagg aagtgtgcaa gaactatgct 960

gaggccaagg atgtcttcct gggcacgttc ttgtatgaat attcaagaag acaccctgat 1020

tactctgtat ccctgttgct gagacttgct aagaaatatg aagccactct ggaaaagtgc 1080
 
tgcgctgaag ccaatcctcc cgcatgctac ggcacagtgc ttgctgaatt tcagcctctt 1140

gtagaagagc ctaagaactt ggtcaaaacc aactgtgatc tttacgagaa gcttggagaa 1200

tatggattcc aaaatgccat tctagttcgc tacacccaga aagcacctca ggtgtcaacc 1260

ccaactctcg tggaggctgc aagaaaccta ggaagagtgg gcaccaagtg ttgtacactt 1320

cctgaagatc agagactgcc ttgtgtggaa gactatctgt ctgcaatcct gaaccgtgtg 1380

tgtctgctgc atgagaagac cccagtgagt gagcatgtta ccaagtgctg tagtggatcc 1440

ctggtggaaa ggcggccatg cttctctgct ctgacagttg atgaaacata tgtccccaaa 1500

gagtttaaag ctgagacctt caccttccac tctgatatct gcacacttcc agagaaggag 1560

aagcagatta agaaacaaac ggctcttgct gagctggtga agcacaagcc caaggctaca 1620

gcggagcaac tgaagactgt catggatgac tttgcacagt tcctggatac atgttgcaag 1680

gctgctgaca aggacacctg cttctcgact gagggtccaa accttgtcac tagatgcaaa 1740

gacgccttag cctaa 1755
<212> Type : DNA
<211> Length : 1755
   Sequence Name : Nucleotide of Murine Albumin domain

### Sequence

<213> Organism Name : human
<400> Pre Sequence String : 
gatgcacaca agagtgaggt tgctcatcgg tttaaagatt tgggagaaga aaatttcaaa 60

gccttggtgt tgattgcctt tgctcagtat cttcagcagt gtccatttga agatcatgta 120

aaattagtga atgaagtaac tgaatttgca aaaacatgtg ttgctgatga gtcagctgaa 180

aattgtgaca aatcacttca tacccttttt ggagacaaat tatgcacagt tgcaactctt 240

cgtgaaacct atggtgaaat ggctgactgc tgtgcaaaac aagaacctga gagaaatgaa 300

tgcttcttgc aacacaaaga tgacaaccca aacctccccc gattggtgag accagaggtt 360
 
gatgtgatgt gcactgcttt tcatgacaat gaagagacat ttttgaaaaa atacttatat 420

gaaattgcca gaagacatcc ttacttttat gccccggaac tccttttctt tgctaaaagg 480

tataaagctg cttttacaga atgttgccaa gctgctgata aagctgcctg cctgttgcca 540

aagctcgatg aacttcggga tgaagggaag gcttcgtctg ccaaacagag actcaagtgt 600

gccagtctcc aaaaatttgg agaaagagct ttcaaagcat gggcagtagc tcgcctgagc 660

cagagatttc ccaaagctga gtttgcagaa gtttccaagt tagtgacaga tcttaccaaa 720

gtccacacgg aatgctgcca tggagatctg cttgaatgtg ctgatgacag ggcggacctt 780

gccaagtata tctgtgaaaa tcaagattcg atctccagta aactgaagga atgctgtgaa 840

aaacctctgt tggaaaaatc ccactgcatt gccgaagtgg aaaatgatga gatgcctgct 900

gacttgcctt cattagctgc tgattttgtt gaaagtaagg atgtttgcaa aaactatgct 960

gaggcaaagg atgtcttcct gggcatgttt ttgtatgaat atgcaagaag gcatcctgat 1020

tactctgtcg tgctgctgct gagacttgcc aagacatatg aaaccactct agagaagtgc 1080

tgtgccgctg cagatcctca tgaatgctat gccaaagtgt tcgatgaatt taaacctctt 1140

gtggaagagc ctcagaattt aatcaaacaa aattgtgagc tttttgagca gcttggagag 1200

tacaaattcc agaatgcgct attagttcgt tacaccaaga aagtacccca agtgtcaact 1260

ccaactcttg tagaggtctc aagaaaccta ggaaaagtgg gcagcaaatg ttgtaaacat 1320

cctgaagcaa aaagaatgcc ctgtgcagaa gactatctat ccgtggtcct gaaccagtta 1380

tgtgtgttgc atgagaaaac gccagtaagt gacagagtca ccaaatgctg cacagaatcc 1440

ttggtgaaca ggcgtccatg cttttcagct ctggaagtcg atgaaacata cgttcccaaa 1500

gagtttaatg ctgaaacatt caccttccat gcagatatat gcacactttc tgagaaggag 1560

agacaaatca agaaacaaac tgcacttgtt gagctcgtga aacacaagcc caaggcaaca 1620

aaagagcaac tgaaagctgt tatggatgat ttcgcagctt ttgtagagaa gtgctgcaag 1680

gctgacgata aggagacctg ctttgccgag gagggtaaaa aacttgttgc tgcaagtcaa 1740
 gctgccttag gcttataa 1758
<212> Type : DNA
<211> Length : 1758
   Sequence Name: nucleotide of human albumin domain

### Organization Applicant

Street : Via S.G. Cottolengo 15
   City : MILAN
   State : MI
   Country : ITALY
   PostalCode : 20143
   PhoneNumber :
   FaxNumber :
   EmailAddress :
   <110> OrganizationName : MEDIOLANUM FARMACEUTICI S.p.A.

### Application Project

<120> Title : A SOLUBLE CHIMERIC INTERLEUKIN 10 RECEPTOR AND THERAPEUTIC USE THEREOF.
<130> AppFileReference : P05076PCT
<140> CurrentAppNumber :
<141> CurrentFilingDate :

### Sequence

<213> OrganismName : human
<400> PreSequenceString : 
MLPCLVVLLA ALLSLRLGSD AHGTELPSPP SVWFEAEFFH HILHWTPIPN QSESTCYEVA
60
LLRYGIESWN SISNCSQTLS YDLTAVTLDL YHSNGYRARV RAVDGSRHSN WTVTNTRFSV
120
DEVTLTVGSV NLEIHNGFIL GKIQLPRPKM APANDTYESI FSHFREYEIA IRKVPGNFTF
180
THKKVKHENF SLLTSGEVGE FCVQVKPSVA SRSNKGMWSK EECISLTRQY FTVTNSRV
238
<212> Type : PRT
   <211> Length : 238
   Sequence Name : human IL10 R alpha extracellular domain

### Sequence

<213> OrganismName : human
<400> PreSequenceString :
   EPKSCDKTHT CPAAA 15
<212> Type : PRT
   <211> Length : 15
   Sequence Name : Human HINGE

### Sequence

<213> OrganismName : human
<400> PreSequenceString : 

60

120

180
 

240

300

360

420

480

540

585
<212> Type : PRT
   <211> Length : 585
   SequenceName : Human albumin domain

### Sequence

<213> OrganismName : human
<400> PreSequenceString : 
atgctgccgt gcctcgtagt gctgctggcg gcgctcctca gcctccgtct tggctcagac
60
gctcatggga cagagctgcc cagccctccg tctgtgtggt ttgaagcaga atttttccac
120
cacatcctcc actggacacc catcccaaat cagtctgaaa gtacctgcta tgaagtggcg
180
ctcctgaggt atggaataga gtcctggaac tccatctcca actgtagcca gaccctgtcc
240
tatgacctta ccgcagtgac cttggacctg taccacagca atggctaccg ggccagagtg
300
cgggctgtgg acggcagccg gcactccaac tggaccgtca ccaacacccg cttctctgtg
360
gatgaagtga ctctgacagt tggcagtgtg aacctagaga tccacaatgg cttcatcctc
420
gggaagattc agctacccag gcccaagatg gcccccgcga atgacacata tgaaagcatc
480
ttcagtcact tccgagagta tgagattgcc attcgcaagg tgccgggaaa cttcacgttc
540
acacacaaga aagtaaaaca tgaaaacttc agcctcctaa cctctggaga agtgggagag
600
ttctgtgtcc aggtgaaacc atctgtcgct tcccgaagta acaaggggat gtggtctaaa
660
gaggagtgca tctccctcac caggcagtat ttcaccgtga ccaactctag agtt
714
<212> Type : DNA
   <211> Length : 714
   Sequence Name : Nucleotide of human IL10 R alpha extracellular domain

### Sequence

<213> OrganismName : human
<400> PreSequenceString : 
gagcccaaat cttgtgacaa aactcacaca tgcccagcgg ccgcg
45
<212> Type : DNA
   <211> Length : 45 SequenceName : Nucleotide of human HINGE

### Sequence

<213> OrganismName : mouse
<400> PreSequenceString : 
MLSRLLPFLV TISSLSLEFI AYGTELPSPS YVWFEARFFQ HILHWKPIPN QSESTYYEVA
60
LKQYGNSTWN DIHICRKAQA LSCDLTTFTL DLYHRSYGYR ARVRAVDNSQ YSNWTTTETR
120
FTVDEVILTV DSVTLKAMDG IIYGTIHPPR PTITPAGDEY EQVFKDLRVY KISIRKFSEL
180
KNATKRVKQE TFTLTVPIGV RKFCVKVLPR LESRINKAEW SEEQCLLITT EQYFTVTNLS
240
IKLI
 244
<212> Type : PRT
   <211> Length : 244
   SequenceName : Mouse IL10 R alpha extracellular domain

### Sequence

<213> OrganismName : mouse
<400> PreSequenceString : 
VPRDCGCKPC ICTGGR
16
<212> Type : PRT
   <211> Length : 16
   SequenceName : mouse hinge

### Sequence

<213> OrganismName : mouse
<400> PreSequenceString : 
EAHKSEIAHR YNDLGEQHFK GLVLIAFSQY LQKCSYDEHA KLVQEVTDFA KTCVADESAA
60
NCDKSLHTLF GDKLCAIPNL RENYGELADC CTKQEPERNE CFLQHKDDNP SLPPFERPEA
120
EAMCTSFKEN PTTFMGHYLH EVARRHPYFY APELLYYAEQ YNEILTQCCA EADKESCLTP
180
KLDGVKEKAL VSSVRQRMKC SSMQKFGERA FKAWAVARLS QTFPNADFAE ITKLATDLTK
240
VNKECCHGDL LECADDRAEL AKYMCENQAT ISSKLQTCCD KPLLKKAHCL SEVEHDTMPA
300
DLPAIAADFV EDQEVCKNYA EAKDVFLGTF LYEYSRRHPD YSVSLLLRLA KKYEATLEKC
360
CAEANPPACY GTVLAEFQPL VEEPKNLVKT NCDLYEKLGE YGFQNAILVR YTQKAPQVST
420
PTLVEAARNL GRVGTKCCTL PEDQRLPCVE DYLSAILNRV CLLHEKTPVS EHVTKCCSGS
480
 
LVERRPCFSA LTVDETYVPK EFKAETFTFH SDICTLPEKE KQIKKQTALA ELVKHKPKAT
540
AEQLKTVMDD FAQFLDTCCK AADKDTCFST EGPNLVTRCK DALA
584
<212> Type : PRT
   <211> Length : 584
   SequenceName : Murine Albumin domain

### Sequence

<213> OrganismName : mouse
<400> PreSequenceString : 
atgttgtcgc gtttgctccc attcctcgtc acgatctcca gcctgagcct agaattcatt
60
gcatacggga cagaactgcc aagcccttcc tatgtgtggt ttgaagccag atttttccag
120
cacatcctcc actggaaacc tatcccaaac cagtctgaga gcacctacta tgaagtggcc
180
ctcaaacagt acggaaactc aacctggaat gacatccata tctgtagaaa ggctcaggca
240
ttgtcctgtg atctcacaac gttcaccctg gatctgtatc accgaagcta tggctaccgg
300
gccagagtcc gggcagtgga caacagtcag tactccaact ggaccaccac tgagactcgc
360
ttcacagtgg atgaagtgat tctgacagtg gatagcgtga ctctgaaagc aatggacggc
420
atcatctatg ggacaatcca tccccccagg cccacgataa cccctgcagg ggatgagtac
480
gaacaagtct tcaaggatct ccgagtttac aagatttcca tccggaagtt ctcagaacta
540
aagaatgcaa ccaagagagt gaaacaggaa accttcaccc tcacggtccc cataggggtg
600
agaaagtttt gtgtcaaggt gctgccccgc ttggaatccc gaattaacaa ggcagagtgg
660
tcggaggagc agtgtttact tatcacgacg gagcagtatt tcactgtgac caacctgagc
720
atcaagctta tt
732
<212> Type : DNA
   <211> Length : 732
   SequenceName : Nucleotide of mouse IL10 R alfa extracellular domain

### Sequence

<213> OrganismName : mouse
<400> PreSequenceString : 
gtgcccaggg attgtggttg taagccttgc atatgtacag gcggccgc
48
<212> Type : DNA
   <211> Length : 48
   SequenceName : Nucleotide of mouse hinge

### Sequence

<213> OrganismName : mouse
<400> PreSequenceString : 
gaagcacaca agagtgagat cgcccatcgg tataatgatt tgggagaaca acatttcaaa
60
ggcctagtcc tgattgcctt ttcccagtat ctccagaaat gctcatacga tgagcatgcc
120
aaattagtgc aggaagtaac agactttgca aagacgtgtg ttgccgatga gtctgccgcc
180
aactgtgaca aatcccttca cactcttttt ggagataagt tgtgtgccat tccaaacctc
240
cgtgaaaact atggtgaact ggctgactgc tgtacaaaac aagagcccga aagaaacgaa
300
tgtttcctgc aacacaaaga tgacaacccc agcctgccac catttgaaag gccagaggct
360
gaggccatgt gcacctcctt taaggaaaac ccaaccacct ttatgggaca ctatttgcat
420
gaagttgcca gaagacatcc ttatttctat gccccagaac ttctttacta tgctgagcag
480
tacaatgaga ttctgaccca gtgttgtgca gaggctgaca aggaaagctg cctgaccccg
540
aagcttgatg gtgtgaagga gaaagcattg gtctcatctg tccgtcagag aatgaagtgc
600
tccagtatgc agaagtttgg agagagagct tttaaagcat gggcagtagc tcgtctgagc
660
cagacattcc ccaatgctga ctttgcagaa atcaccaaat tggcaacaga cctgaccaaa
720
gtcaacaagg agtgctgcca tggtgacctg ctggaatgcg cagatgacag ggcggaactt
780
gccaagtaca tgtgtgaaaa ccaggcgact atctccagca aactgcagac ttgctgcgat
840
aaaccactgt tgaagaaagc ccactgtctt agtgaggtgg agcatgacac catgcctgct
900
gatctgcctg ccattgctgc tgattttgtt gaggaccagg aagtgtgcaa gaactatgct
960
gaggccaagg atgtcttcct gggcacgttc ttgtatgaat attcaagaag acaccctgat
1020
tactctgtat ccctgttgct gagacttgct aagaaatatg aagccactct ggaaaagtgc
1080
tgcgctgaag ccaatcctcc cgcatgctac ggcacagtgc ttgctgaatt tcagcctctt
1140
gtagaagagc ctaagaactt ggtcaaaacc aactgtgatc tttacgagaa gcttggagaa
1200
tatggattcc aaaatgccat tctagttcgc tacacccaga aagcacctca ggtgtcaacc
1260
ccaactctcg tggaggctgc aagaaaccta ggaagagtgg gcaccaagtg ttgtacactt
1320
cctgaagatc agagactgcc ttgtgtggaa gactatctgt ctgcaatcct gaaccgtgtg
1380
tgtctgctgc atgagaagac cccagtgagt gagcatgtta ccaagtgctg tagtggatcc
1440
ctggtggaaa ggcggccatg cttctctgct ctgacagttg atgaaacata tgtccccaaa
1500
gagtttaaag ctgagacctt caccttccac tctgatatct gcacacttcc agagaaggag
1560
aagcagatta agaaacaaac ggctcttgct gagctggtga agcacaagcc caaggctaca
1620
gcggagcaac tgaagactgt catggatgac tttgcacagt tcctggatac atgttgcaag
1680
 
gctgctgaca aggacacctg cttctcgact gagggtccaa accttgtcac tagatgcaaa
1740
gacgccttag cctaa
1755
<212> Type : DNA
   <211> Length : 1755
   SequenceName : Nuucleotide of Murine Albumin domain

### Sequence

<213> OrganismName : human
<400> PreSequenceString : 
gatgcacaca agagtgaggt tgctcatcgg tttaaagatt tgggagaaga aaatttcaaa
60
gccttggtgt tgattgcctt tgctcagtat cttcagcagt gtccatttga agatcatgta
120
aaattagtga atgaagtaac tgaatttgca aaaacatgtg ttgctgatga gtcagctgaa
180
aattgtgaca aatcacttca tacccttttt ggagacaaat tatgcacagt tgcaactctt
240
cgtgaaacct atggtgaaat ggctgactgc tgtgcaaaac aagaacctga gagaaatgaa
300
tgcttcttgc aacacaaaga tgacaaccca aacctccccc gattggtgag accagaggtt
360
gatgtgatgt gcactgcttt tcatgacaat gaagagacat ttttgaaaaa atacttatat
420
gaaattgcca gaagacatcc ttacttttat gccccggaac tccttttctt tgctaaaagg
480
tataaagctg cttttacaga atgttgccaa gctgctgata aagctgcctg cctgttgcca
540
aagctcgatg aacttcggga tgaagggaag gcttcgtctg ccaaacagag actcaagtgt
600
gccagtctcc aaaaatttgg agaaagagct ttcaaagcat gggcagtagc tcgcctgagc
660
cagagatttc ccaaagctga gtttgcagaa gtttccaagt tagtgacaga tcttaccaaa
720
gtccacacgg aatgctgcca tggagatctg cttgaatgtg ctgatgacag ggcggacctt
780
gccaagtata tctgtgaaaa tcaagattcg atctccagta aactgaagga atgctgtgaa
840
aaacctctgt tggaaaaatc ccactgcatt gccgaagtgg aaaatgatga gatgcctgct
900
gacttgcctt cattagctgc tgattttgtt gaaagtaagg atgtttgcaa aaactatgct
960
gaggcaaagg atgtcttcct gggcatgttt ttgtatgaat atgcaagaag gcatcctgat
1020
tactctgtcg tgctgctgct gagacttgcc aagacatatg aaaccactct agagaagtgc
1080
tgtgccgctg cagatcctca tgaatgctat gccaaagtgt tcgatgaatt taaacctctt
1140
gtggaagagc ctcagaattt aatcaaacaa aattgtgagc tttttgagca gcttggagag
1200
tacaaattcc agaatgcgct attagttcgt tacaccaaga aagtacccca agtgtcaact
1260
ccaactcttg tagaggtctc aagaaaccta ggaaaagtgg gcagcaaatg ttgtaaacat
1320
 
cctgaagcaa aaagaatgcc ctgtgcagaa gactatctat ccgtggtcct gaaccagtta
1380
tgtgtgttgc atgagaaaac gccagtaagt gacagagtca ccaaatgctg cacagaatcc
1440
ttggtgaaca ggcgtccatg cttttcagct ctggaagtcg atgaaacata cgttcccaaa
1500
gagtttaatg ctgaaacatt caccttccat gcagatatat gcacactttc tgagaaggag
1560
agacaaatca agaaacaaac tgcacttgtt gagctcgtga aacacaagcc caaggcaaca
1620
aaagagcaac tgaaagctgt tatggatgat ttcgcagctt ttgtagagaa gtgctgcaag
1680
gctgacgata aggagacctg ctttgccgag gagggtaaaa aacttgttgc tgcaagtcaa
1740
gctgccttag gcttataa
1758
<212> Type : DNA
   <211> Length : 1758
   SequenceName : nucleotide of human albumin domain

## Claims

1. A chimeric fusion protein comprising albumin and the extracellular domain of the alpha unit of the interleukin 10 (IL10) receptor.

2. The chimeric fusion protein according to claim 1, wherein said albumin is a mammalian serum albumin.

3. The chimeric fusion protein according to claim 2, wherein said mammalian serum is human or murine.

4. The chimeric fusion protein according to any one of the claims 1-3, wherein said extracellular domain of the alpha unit of the IL10 receptor comes from mammalian peripheral blood cells (PBMCs).

5. The chimeric fusion protein according to claim 4, wherein said PBMCs are human or murine.

6. The chimeric fusion protein according to any one of the claims 1-5, wherein the albumin is bonded to said extracellular domain of the alpha unit of the IL10 receptor by means of a spacer.

7. The chimeric protein according to claim 6, wherein said spacer is the hinge region of an immuno-gamma globulin G (IgG).

8. The chimeric fusion protein according to claim 7, wherein said IgG is IgG1.

9. The chimeric fusion protein according to claim 7 or 8, wherein said IgG comes from mammalian PBMCs.

10. The chimeric fusion protein according to claim 9, wherein said PBMCs are human or murine.

11. A polynucleotide construct that encodes for the chimeric fusion protein according to any one of the claims 1-10.

12. The polynucleotide construct according to claim 11, being a gene.

13. The polynucleotide construct according to claim 12, wherein said gene is comprised in a vector.

14. The polynucleotide construct according to claim 13, wherein said vector is a plasmid.

15. The chimeric fusion protein according to any one of the claims 1-10, for use in the treatment of IL10 correlated pathologies.

16. The chimeric protein for use according to claim 15, wherein said pathologies are cancer and systemic lupus erythematosus (SLE).

17. The polynucleotide construct according to any one of the claims 11-14, for use in the treatment of IL10 correlated pathologies.

18. The polynucleotide construct for use according to claim 17, wherein said pathologies are cancer and SLE.

## Patentansprüche

1. Chimäres Fusionsprotein, umfassend Albumin und die extrazelluläre Domäne der Alpha-Einheit des Interleukin 10 (IL10) -Rezeptors.

2. Chimäres Fusionsprotein nach Anspruch 1, wobei das Albumin ein Säuger-Serumalbumin ist.

3. Chimäres Fusionsprotein nach Anspruch 2, wobei das Säuger-Serum human oder murin ist.

4. Chimäres Fusionsprotein nach einem der Ansprüche 1 bis 3, wobei die extrazelluläre Domäne der Alpha-Einheit des IL10-Rezeptors aus peripheren Säugerblutzellen (PBMC) stammt.

5. Chimäres Fusionsprotein nach Anspruch 4, wobei die PBMC human oder murin sind.

6. Chimäres Fusionsprotein nach einem der Ansprüche 1 bis 5, wobei das Albumin mittels eines Abstandhalters an die extrazelluläre Domäne der Alpha-Einheit des IL10-Rezeptors gebunden ist.

7. Chimäres Protein nach Anspruch 6, wobei der Abstandhalter die Gelenkregion eines Immuno-Gamma-Globulins G (IgG) ist.

8. Chimäres Fusionsprotein nach Anspruch 7, wobei das IgG IgG1 ist.

9. Chimäres Fusionsprotein nach Anspruch 7 oder 8, wobei das IgG aus 2-r-Säuger-PBMC stammt.

10. Chimäres Fusionsprotein nach Anspruch 9, wobei die PBMC human oder murin sind.

11. Polynukleotidkonstrukt, das für das chimäre Fusionsprotein nach einem der Ansprüche 1 bis 10 kodiert.

12. Polynukleotidkonstrukt nach Anspruch 11, wobei es sich um ein Gen handelt.

13. Polynukleotidkonstrukt nach Anspruch 12, wobei das Gen in einem Vektor enthalten ist.

14. Polynukleotidkonstrukt nach Anspruch 13, wobei der Vektor ein Plasmid ist.

15. Chimäres Fusionsprotein nach einem der Ansprüche 1 bis 10 zur Verwendung bei der Behandlung von mit IL10 korrelierten Pathologien.

16. Chimäres Protein zur Verwendung nach Anspruch 15, wobei die Pathologien Krebs und systemischer Lupus erythematodes (SLE) sind.

17. Polynukleotidkonstrukt nach einem der Ansprüche 11-14 zur Verwendung bei der Behandlung von mit IL10 korrelierten Pathologien.

18. Polynukleotidkonstrukt zur Verwendung nach Anspruch 17, wobei die Pathologien Krebs und SLE sind.

## Revendications

1. Une protéine de fusion chimérique comprenant l'albumine et le domaine extracellulaire de l'unité alpha du récepteur de l'interleukine 10 (IL10).

2. Protéine de fusion chimérique selon la revendication 1, dans laquelle ladite albumine est une albumine sérique de mammifère.

3. Protéine de fusion chimérique selon la revendication 2, dans laquelle ledit sérum de mammifère est humain ou murin.

4. Protéine de fusion chimérique selon l'une quelconque des revendications 1 à 3, dans laquelle ledit domaine extracellulaire de l'unité alpha du récepteur de l'IL10 provient de cellules sanguines périphériques de mammifère (PBMC).

5. Protéine de fusion chimérique selon la revendication 4, dans laquelle lesdites PBMC sont humaines ou murines.

6. Protéine de fusion chimérique selon l'une quelconque des revendications 1 à 5, dans laquelle l'albumine est liée audit domaine extracellulaire de l'unité alpha du récepteur de l'IL10 au moyen d'un espaceur.

7. Protéine chimérique selon la revendication 6, dans laquelle ledit espaceur est la région charnière d'une immuno-gamma globuline G (IgG).

8. Protéine de fusion chimérique selon la revendication 7, dans laquelle ladite IgG est une IgG1.

9. Protéine de fusion chimérique selon la revendication 7 ou 8, dans laquelle ladite IgG provient de 2-r PBMC de mammifère.

10. Protéine de fusion chimérique selon la revendication 9, dans laquelle lesdites PBMC sont humaines ou murines.

11. Une construction polynucléotidique qui code pour la protéine de fusion chimérique selon l'une quelconque des revendications 1 à 10.

12. Construction polynucléotidique selon la revendication 11, qui est un gène.

13. Construction polynucléotidique selon la revendication 12, dans laquelle ledit gène est compris dans un vecteur.

14. Construction polynucléotidique selon la revendication 13, dans laquelle ledit vecteur est un plasmide.

15. Protéine de fusion chimérique selon l'une quelconque des revendications 1 à 10, destinée à être utilisée dans le traitement de pathologies corrélées à l'IL10.

16. Protéine chimérique à utiliser selon la revendication 15, dans laquelle lesdites pathologies sont le cancer et le lupus érythémateux systémique (LES).

17. Construction polynucléotidique selon l'une quelconque des revendications 11 à 14, destinée à être utilisée dans le traitement de pathologies corrélées à l'IL10.

18. Construction polynucléotidique à utiliser selon la revendication 17, dans laquelle lesdites pathologies sont le cancer et le LES.
